# EUROPÄISCHE PATENTSCHRIFT

(11) **EP 0 416 499 B1**
(45) Veröffentlichungstag und Bekanntmachung des Hinweises auf die Patenterteilung: **26.04.1995**
(21) Anmeldenummer: 90116859.1
(22) Anmeldetag: 03.09.1990
(51) Int. Cl.: C07D 295/195, C07C 279/22, A61K 31/445, A61K 31/155

(54) **Benzoylguanidine, Verfahren zu ihrer Herstellung, ihre Verwendung als Medikament sowie sie enthaltendes Medikament**
Benzoylguanidines, process for their preparation, their use as medicaments as well as medicament containing them
Benzoylguanidines, procédé pour leur préparation, leur utilisation comme médicaments ainsi que médicaments les contenant

(30) Priorität: 06.09.1989 DE 3929582
(43) Veröffentlichungstag der Anmeldung: 13.03.1991
(73) Patentinhaber: HOECHST AKTIENGESELLSCHAFT, 65926 Frankfurt am Main (DE)
(72) Erfinder: Englert, Heinrich Christian, Dr., D-6238 Hofheim am Taunus (DE); Lang, Hans-Jochen, Dr., D-6238 Hofheim am Taunus (DE); Linz, Wolfgang, Dr., D-6500 Mainz (DE); Schölkens, Bernward, Prof. Dr., D-6233 Kelkheim(Taunus) (DE); Scholz, Wolfgang, Dr., D-6236 Eschborn (DE)

(56) Entgegenhaltungen:
- DE-A- 2 643 952
- US-A- 3 432 551
- US-A- 4 178 387
- US-A- 4 380 027
- JOURNAL OF MEDICINAL CHEMISTRY, Band 32, Nr. 8, August 1989, Seiten 1963-1970; A. BUSCHAUER: "Synthesis and in Vitro Pharmacology of Arpromidine and related Phenyl(pyridylakyl) guanidines, a Potential New Class of Positive Inotropic Drugs"
- CIRCULATION,Band 79, Nr. 6, Juni 1989, Seiten 1257-1263; H.J. DUFF et al.: "Amiloride. Antiarrhythmic and Electrophysiologic Actions in Patients with Inducible Sustained Ventricular Tachycardia"

## Beschreibung

Die Erfindung betrifft Benzoylguanidine der Formel I 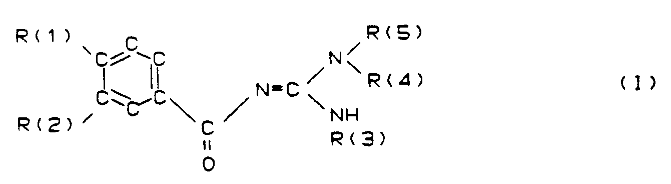
worin R(1) oder R(2) R(6)-S(O)ₙ- oder 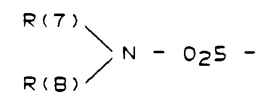
bedeuten
und der jeweils andere Substituent R(1) oder R(2)
H, F, Cl, Br, J, C₁-C₄-Alkyl, C₁-C₄-Alkoxy, Phenoxy, das unsubstituiert ist oder einen bis drei Substituenten aus den Gruppen Fluor, Chlor, Methyl oder Methoxy trägt, R(6)-S(O)ₙ oder 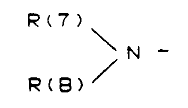
bedeutet,
und worin R⁶ für C₁-C₆-Alkyl, C₅-C₇-Cycloalkyl, Cyclopentylmethyl, Cyclohexylmethyl oder für Phenyl steht, das unsubstituiert ist oder einen bis drei Substituenten aus den Gruppen Fluor, Chlor, Methyl oder Methoxy trägt,
und worin R(7) und R(8) gleich oder verschieden sind und in der Bedeutung von H, C₁-C₆-Alkyl stehen oder R(7) für Phenyl-(CH₂)ₘ- mit m=1-4 oder Phenyl steht, das unsubstituiert ist oder einen bis zwei Substituenten aus den Gruppen Fluor, Chlor, Methyl oder Methoxy trägt,
und worin R(7) und R(8) auch gemeinsam eine geradkettige oder verzweigte C₄-C₇-Kette sein können, wobei die Kette zusätzlich durch O, S oder NR(9) unterbrochen sein kann und R(9) für H oder Methyl steht und worin R(7) und R(8) gemeinsam mit dem Stickstoffatom, an das sie gebunden sind, ein Dihydroindol, Tetrahydrochinolin oder Tetrahydroisochinolin-System bedeuten können,
und R(3), R(4) und R(5) Wasserstoff, C₁-C₂-Alkyl bedeuten, oder wobei entweder R(3) und R(4) gemeinsam eine (C₂-C₄)-Alkylenkette oder R(4) und R(5) gemeinsam eine (C₄-C₇)-Alkylenkette bilden können,
und n für Null, 1 oder 2 steht,
sowie deren pharmazeutisch verträgliche Salze.

Enthalten die Substituenten R(1) bis R(5) ein oder mehrere Asymmtriezentren, so gehören sowohl S als auch R konfigurierte Verbindungen zur Erfindung. Die Verbindungen können als optische Isomere, als Diastereoisomere, als Racemate oder als Gemische derselben vorliegen.
Die bezeichneten Alkylreste können sowohl geradkettig wie verzweigt vorliegen.

Bevorzugt sind Verbindungen der Formel I, worin
R(1) Fluor, Chlor, NR(7)R(8), R(6)-S(O)ₙ- oder Phenoxy bedeutet,
R(2) für R(6)-S(O)ₙ- und R(7)R(8)N-SO₂- steht
und worin n Null, 1, 2 ist
und R(3),R(4) und R(5) Wasserstoff bedeuten,
und worin R(6), R(7) und R(8) die oben angegebene Bedeutung haben und deren pharmakologisch verträgliche Salze.

Besonders bevorzugt sind Verbindungen der Formel I mit R(1) in der Bedeutung von R(7)R(8)N- oder R(6)-S(O)ₙ-, worin R(6) Alkyl mit 1-3 C-Atomen oder Phenyl bedeuten, n=Null und R(7) und R(8) Wasserstoff, Alkyl mit 1-4 C-Atomen oder R(7) und R(8) gemeinsam eine (C₄-C₇)-Methylenkette bilden und
R(2) für CH₃-SO₂- oder H₂N-SO₂- steht, R(3), R(4) und R(5) gleich Wasserstoff und deren pharmakologisch verträgliche Salze.

Ganz besonders bevorzugt sind 3-Methylsulfonyl-4-(1-piperidinyl)- benzoyl-guanidin, 4-Phenylthio-3-sulfamoylbenzoyl-guanidin und 3-Methylsulfonyl-4-phenylthiobenzoyl-guanidin und deren pharmakologisch verträgliche Salze.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 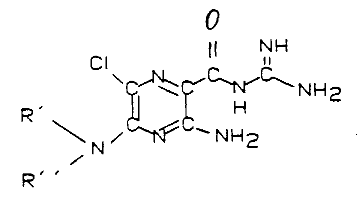
Amilorid: R',R'' = H
Dimethylamilorid: R',R'' = CH₃
Ethylisopropylamilorid: R' = C₂H₅, R'' = CH(CH₃)₂
Darüberhinaus sind Untersuchungen bekannt geworden, die auf antiarrhythmische Eigenschaften von Amilorid hinweisen (Circulation 79, 1257-63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts)). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

Im US-Patent 3,780,027 werden Acylguanidine beansprucht, die strukturell den Verbindungen der Formel I ähnlich sind. Der entscheidende Unterschied zu den Verbindungen I besteht darin, daß es sich um trisubstituierte Benzoylguanidine handelt, die sich in ihrem Substitutionsmuster von im Handel befindlichen Diuretika, wie Bumetanid und Furosemid, ableiten und eine für die angestrebte salidiuretische Wirkung wichtige Aminogruppe in Position 2 bzw. 3 zur Carbonylguanidingruppe tragen. Entsprechend wird für diese Verbindungen eine starke salidiuretische Wirksamkeit berichtet.

Es war daher sehr überraschend, daß die erfindungsgemäßen Verbindungen keine unerwünschten salidiuretischen, jedoch sehr gute antiarrhythmische Eigenschaften aufweisen. Die Verbindungen sind infolge ihrer pharmakologischen Eigenschaften als antiarrhytmische Arzneimittel mit cardioprotektiver Komponente zur Infarktprophylaxe und der Infarktbehandlung sowie zur Behandlung der angina pectoris hervorragend geeignet, wobei sie auch präventiv eingesetzt werden können.

Die Erfindung betrifft weiterhin ein Verfahren zur Herstellung der Verbindungen I, dadurch gekennzeichnet, daß man Verbindungen der Formel II 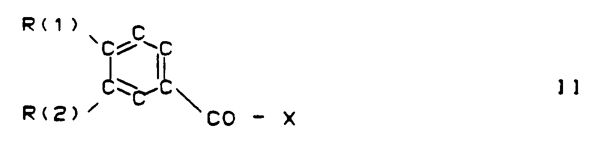
mit einem Guanidinderivat der Formel III 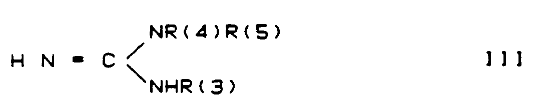
umsetzt, worin R(1) bis R(5) die angegebene Bedeutung besitzen und X für eine leicht nucleophil substituierbare leaving group steht.

Die aktivierten Säurederivate der Formel II, worin X eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, X=Cl), die man ihrerseits wiederum in an sich bekannter Weise aus den zugrundeliegenden Carbonsäuren (Formel II, X= OH) beispielsweise mit Thionylchlorid herstellen kann.
Neben den Carbonsäurechloriden der Formel II (X=Cl) lassen sich auch weitere aktivierte Säurederivate der Formel II in an sich bekannter Weise direkt aus den zugrundeliegenden Benzoesäure-derivaten (Formel II, X = OH) herstellen, wie beispielsweise die Methylester der Formel II mit X=OCH₃ durch Behandeln mit gasförmigem HCl in Methanol, die Imidazolide der Formel II durch Behandeln mit Carbonyldiimidazol (X= 1-Imidazolyl, Staab, Angew. Chem. Int. Ed. Engl. 1,351-367 (1962)), die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC). Eine Reihe geeigneter Methoden zur Herstellung von aktivierten Carbonsäure-derivaten der Formel II sind unter Angabe von Quellenliteratur in J. March, Advanced Organic Chemistry, Third Edition (John Wiley&Sons, 1985), S. 350 angegeben.
Eine weitere Methode zur Darstellung insbesondere von solchen Verbindungen der Formel I, in denen R(4) und/oder R(5) nicht H bedeuten, besteht in der Umsetzung eines Säurechlorides oder anderen geeigneten aktivierten Benzoesäurederivates der Formel II mit 3,5-Dimethylpyrazol-1-carbonsäureamidin-nitrat (erhältlich bei Aldrich) in Gegenwart von Natronlauge und nachfolgender Behandlung des N-Benzoyl-3,5-dimethylpyrazol-1-carbonsäureamidin-Derivates mit Ammoniak oder einem Amin HNR(4)R(5) (Methods Enzymol.,25 b, 558 (1972).

Die Umsetzung eines aktivierten Carbonsäurederivates der Formel I mit einem Guanidinderivat der Formel III erfolgt in an sich bekannter Weise in einem protischen oder aprotischen polaren aber inerten organischen Lösungsmittel. Dabei haben sich bei der Umsetzung der Benzoesäuremethylester (II, X=OMe)mit Guanidin Methanol oder THF zwischen 20°C und Siedetemperatur dieser Lösungsmittel bewährt. Bei den meisten Umsetzungen von Verbindungen II mit salzfreien Guanidinen III wurde vorteilhaft in aprotischen inerten Lösungsmitteln wie THF, Dimethoxyethan, Dioxan gearbeitet. Aber auch Wasser kann als Lösungsmittel bei der Umsetzung von II und III verwendet werden.

Wenn X=Cl bedeutet, arbeitet man vorteilhaft unter Zusatz eines Säurefängers, z.B. in Form von überschüssigen Guanidin zur Abbindung der Halogenwasserstoffsäure.

Ein Teil der zugrundeliegenden Benzoesäurederivate der Formel II und die verwendeten Guanidine der Formel III sind bekannt und in der Literatur beschrieben. Die unbekannten Verbindungen der Formel II können nach literaturbekannten Methoden hergestellt werden, indem man beispielsweise 4-Chlor- oder 4-Fluor-3-chlor-sulfonylbenzoesäure mit Ammoniak oder einem Amin in eine 3-Aminosulfonyl-4-chlor- oder -fluorbenzoesäure (IV a) bzw. mit einem schwachen Reduktionsmittel wie Natriumbisulfit und anschließender Alkylierung in eine 3-Alkylsulfonyl-4-chlor- oder -4-fluorbenzoesäure (IV b, n=2) 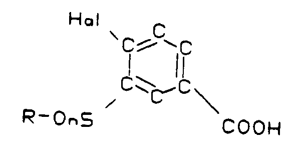
IVa): R = R(6)
IVb): R = NR(7)R(8)
überführt und die erhaltenen Benzoesäuren nach einer der oben beschriebenen Verfahrensvarianten zu erfindungsgemäßen Verbindungen I umgesetzt werden.

Verbindungen der Formeln IVa) und b) können aber auch als Ausgangsverbindungen anderer Carbonsäuren dienen, wobei das Halogen in R(1)-Position sehr bequem gegen zahlreiche nucleophile Reagentien, wie Mercaptane R(6)-SH oder primäre Aminen R(7)R(8)NH unter Bildung weiterer Benzoesäurederivate II mit X= OH ausgetauscht werden kann.
In ähnlicher Weise können ausgehend von 3-Nitro-4-chlorbenzoesäure durch nucleophile Einführung eines erfindungsgemäßen Restes R(1) in Position 4 (Austausch gegen Cl) und weiterer Abwandlung der Nitrogruppe, wie Reduktion zu NH₂ und nachfolgende Alkylierung oder Verdrängung, beispielsweise durch Diazotierung und Sandmeyer-Reaktion, weitere Benzoesäurederivate (II, X=OH) hergestellt werden.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p- Toluolsulfonate.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.
Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg, vorzugsweise 0,01 mg bis höchstens 10 mg, vorzugsweise höchstens 1 mg. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 100 mg pro Tag notwendig werden.

Analog der in den Ausführungsbeispielen angegebenenen Vorschriften können die nachfolgend aufgeführten erfindungsgemäßen Verbindungen der Formel I bzw. deren physiologisch verträglichen Salze hergestellt werden:
4-Methoxy-3-methylsulfonylbenzoyl-guanidin,
4-Isobutyloxy-3-methylsulfonylbenzoyl-guanidin,
4-Benzyloxy-3-methylsulfonylbenzoyl-guanidin,
4-(1-Butylthio)-3-methylsulfonylbenzoyl-guanidin,
4-(1-Butylsulfinyl)-3-methylsulfonylbenzoyl-guanidin,
4-(1-Butylsulfonyl)-sulfamoylbenzoyl-guanidin,
4-(1-Butylsulfonyl)-3-methylsulfonylbenzoyl-guanidin,
4-(2-Chlorphenylthio)-3-methylsulfonylbenzoyl-guanidin,
4-Phenoxy-3-methylsulfonylbenzoyl-guanidin,
4-Cyclohexylthio-3-methylsulfonylbenzoyl-guanidin,
4-Cyclohexylsulfinyl-3-methylsulfonylbenzoyl-guanidin,
4-Cyclohexylsulfonyl-3-methylsulfonylbenzoyl-guanidin,
4-(1-Piperidino-)-3-sulfamoylbenzoyl-guanidin,
4-Methoxy-3-sulfamoylbenzoyl-guanidin,
4-Isobutyloxy-3-sulfamoylbenzoyl-guanidin,
4-Benzyloxy-3-sulfamoylbenzoyl-guanidin,
4-(1-Butylthio)-3-sulfamoylbenzoyl-guanidin,
4-(1-Butylsulfinyl)-3-sulfamoylbenzoyl-guanidin,
4-(2-Chlorphenylthio)-3-sulfamoylbenzoyl-guanidin,
4-Cyclohexylthio)-3-sulfamoylbenzoyl-guanidin,
4-Cyclohexylsulfonyl-3-sulfamoylbenzoyl-guanidin,
3-Isopropylsulfamoyl-4-(1-piperidino)-benzoyl-guanidin,
3-(1-Butylsulfamoyl)-4-(1-piperidino)-benzoyl-guanidin,
3-(N,N-Di-1-butylsulfamoyl)-4-(1-piperidino)-benzoylguanidin,
3-(3-Methoxypropylsulfamoyl)-4-(1-piperidino)-benzoylguanidin,
3-(3,5-Dimethylpiperidino-N-sulfonyl)-4-(1-piperidino)-benzoyl-guanidin,
3-Cyclopentylsulfamoyl-4-(1-piperidino)-benzoyl-guanidin
4-(3-Methoxy-1-propylamino)-3-methylsulfonylbenzoyl-guanidin,
4-(3-Methoxy-1-propylamino)-3-sulfamoylbenzoyl-guanidin,
4-(N,N-Di-1-butylamino)-3-methylsulfonylbenzoyl-guanidin,
4-(N,N-Di-1-butylamino)-3-sulfamoylbenzoyl-guanidin,
4-N-Pentamethylenamino-3-methylsulfonylbenzoyl-guanidin,
4-N-Pentamethylenamino-3-sulfamoylbenzoyl-guanidin,
4-Cyclopentylamino-3-methylsulfonylbenzoyl-guanidin,
4-Cyclopentylamino-3-sulfamoylbenzoyl-guanidin,
4-(2-Chlorphenylamino)-3-methylsulfonylbenzoylguanidin,
4-(2-Chlorphenylamino)-3-sulfamoylbenzoyl-guanidin,
4-(4-Methoxyphenylamino)-3-methylsulfonylbenzoyl-guanidin,
4-(4-Methoxyphenylamino)-3-sulfamoylbenzoyl-guanidin,
4-(N-Methyl-N-phenylamino)-3-methylsulfonylbenzoyl-guanidin,
4-(N-Methyl-N-phenylamino)-3-sulfamoylbenzoyl-guanidin,
4-(2,4-Dimethylamino)-3-methylsulfonylbenzoyl-guanidin,
4-(2,4-Dimethylamino)-3-sulfamoylbenzoyl-guanidin,
4-(3-Methylphenylamino)-3-methylsulfonylbenzoyl-guanidin,
4-(3-Methylphenylamino)-3-sulfamoylbenzoyl-guanidin,
4-(4-Methylphenylthio)-3-sulfamoylbenzoyl-guanidin
4-(4-Chlorphenylthio)-3-sulfamoylbenzoyl-guanidin,
4-(4-Methoxyphenylthio)-3-sulfamoylbenzoyl-guanidin,
4-Methyl-3-methylsulfonylbenzoyl-guanidin,
4-Methyl-3-sulfamoylbenzoyl-guanidin,

### Experimenteller Teil

### Beispiel 1:

### 4-Chlor-3-sulfamoylbenzoyl-guanidin-hydrochlorid

Zu einer methanolischen Natriummethylatlösung, dargestellt aus 1,9 g Natrium in 90 ml Methanol, gibt man 8,5 g Guanidin-hydro-chlorid und anschließend 5 g 4-Chlor-3-sulfamoylbenzoesäure-methylester. Nach Erwärmen dieser Mischung über 30 Stunden unter inertgas (Stickstoff oder Argon) auf 50-60°C destilliert man das Lösungsmittel ab, stellt nach Aufnehmen des Rückstandes mit 2N Essigsäure auf pH 6 und extrahiert mit Essigester. Nach Acidifizieren des getrockneten Extraktes (Trocknung über Magnesiumsulfat) mit etherischer Salzsäure filtriert man die Titelverbindung ab.
Farblose Kristalle, Schmp. 305-308°C.

### Beispiel 2

### 3-N,N-Diethylsulfamoyl-4-chlorbenzoyl-guanidin-hydrochlorid

erhält man analog der in Beispiel 1 angegebenen Vorschrift durch Umsetzung von 0,112 Mol Guanidin, dargestellt aus 10,7 g Guanidin-hydrochlorid und der äquimolekularen Menge an Natriummethylat in 70 ml Methanol.

### Farblose Kristalle, Schmp. 202-205°C

### Beispiel 3

### 4-Chlor-3-methylsulfonylbenzoyl-guanidin-hydrochlorid

erhält man analog der in Beispiel 1 angegebenen Vorschrift durch Umsetzung von 42,2 mMol Guanidin, dargestellt aus 4,03 g Guanidin-hydrochlorid und 7,7 g Natrium-(bis-trimethylsilylamid) durch Erwärmen in Methanol über 6 Stunden auf 50°C und analoge Aufarbeitung.
Farblose Kristalle, Schmp. 220°C (Zers.).

### Beispiel 4:

### 3-Methylsulfonyl-4-(1-piperidyl)-benzoyl-guanidin

a) 5-Carboxy-2-chlorbenzolsulfinsäure Dinatriumsalz
   erhält man aus 186 g 5-Carboxy-2-chlorbenzol-sulfinsäure, dargestellt durch Reduktion von 261 g 5-Carboxy-2-chlorbenzolsulfonsäurechlorid mit 157 g Natriumsulfit in 700 ml Wasser bei 70°C bei pH 9-10 und anschließendes Acidifizieren mit HCl, durch Neutralisation mit 67,6 g Ätznatron in 2 ltr. Wasser, Abdestillieren und Kristallisation des Rückstandes unter Aceton.
   Weißes Kristallpulver, Schmp. >340°C.
b) 4-Chlor-3-methylsulfonylbenzoesäure-methylester
   52,9 g des unter 4a) dargestellten Dinatriumsalzes werden in 400 ml wasserfreiem Dimethylformamid mit 99,3 g Methyljodid über 5 Stunden bei 50-60°C gerührt, das Lösungsmittel abdestilliert und nach Verrühren und mehrfachem Waschen des Rückstandes mit Wasser wird die Substanz abfiltriert.
   Weißes Kristallpulver, Schmp. 150-153°C
c) 4-Chlor-3-methylsulfonylbenzoesäure
   14,3 g des unter 4b) dargestellten Benzoesäuremethylester-Derivates in 120 ml Methanol und 50 ml 2N Natronlauge über 6 Stunden bei Zimmertemperatur gerührt, das Lösungsmittel abdestilliert, nach Auflösung des Rückstandes in Wasser mit 2N Salzsäure auf pH 0-1 gestellt und die Substanz abfiltriert. Weißes Kristallpulver, Schmp. 220-224°C.
d) 3-Methylsulfonyl-4-(1-piperidino)-benzoesäure
   92 g 4-Chlor-3-methylsulfonylbenzoesäure werden in 460 ml Piperidin 11 Stunden am Rückflußkühler gekocht, danach mit 800 ml Wasser verdünnt und mit konz. Salzsäure auf pH 2 gestellt. Nach Kristallisation im Eisbad filtriert man den Feststoff ab.
   Farblose Kristalle aus Ethanol, Schmp. 234-238°C.
e) 3-Methylsulfonyl-4-(1-piperidino)-benzoylchlorid
   erhält man durch Rückflußkochen von 50 g 3-Methylsulfonyl-4-(1- piperidino)-benzoesäure über 4 Stunden in 400 ml Thionylchlorid, sorgfältiges Abdestillieren des Lösungsmittels unter vermindertem Druck und Kristallisation des Rückstandes unter Dimethylether.
f) 3-Methylsulfonyl-4-(1-piperidino)-benzoylguanidin
   21,8 g 3-Methylsulfonyl-4-(1-piperidino)-benzoylchlorid werden mit 31,4 g Guanidin in 200 ml 1,2-Dimethoxyethan unter Inertgas über 2 Stunden bei 5-10°C gerührt, unter Kühlung mit ca. 200 ml Wasser versetzt und mit 2N HCl auf pH 6-7 gestellt.
   Nach Abdestillieren der Hälfte des Lösungsvolumens filtriert man die Kristalle ab und kristallisiert aus Methanol um.
   Farblose Kristalle, Schmp. 210°-213°C (Zers.)

### Beispiel 5:

### 3-Methylsulfonyl-4-(1-piperidino)-benzoylguanidin-dihydrochlorid

erhält man aus 3-Methylsulfonyl-4-(1-piperidino)-benzoylguanidin in der 10 fachen Gewichtsmenge Methanol durch Zugabe von überschüssiger etherischer HCl und Rührung des Salzniederschlages unter Kühlung im Eisbad.
Weißes Kristallpulver, Schmp. 210-214°C (Zers.).

### Beispiel 6:

### 3-Methylsulfonyl-4-(1-piperidino)-benzoylguanidin-hydrochlorid

erhält man durch Behandeln von 200 g des Dihydrochlorides (Beispiel 5) mit der äquivalenten Menge einer monovalenten Base (NaOH, NaHCO₃, N(C₂H₅)₃ usw.) in 1,3 bis 1,4 ltr. Wasser oder durch einfaches Umkristallisieren der angegebenen Substanzmenge aus 1,3- 1,4 ltr. Wasser.
Farblose Kristalle, Schmp. 235-237°C.

### Beispiel 7:

### 4-Chlor-3-N,N-Dimethylsulfamoylbenzoyl-guanidin-hydrochlorid

erhält man analog der in Beispiel 1 angegebenen Vorschrift durch Umsetzung von 2,77 g (0,01 Mol) 4-Chlor-3-dimethylsulfamoyl-benzoesäure-methylester (Schmp. 95-96°C) mit 0,04 Mol Guanidin in Methanol und anschließender analoger Aufarbeitung.
Weißes Kristallpulver, Schmp. 224-226°C (Zers.)

### Beispiel 8

### 4-Chlor-3-(1-piperidyl-sulfonyl)-benzoylguanidin-hydrochlorid

erhält man analog der in Beispiel 1 angegebenen Vorschrift aus 4-Chlor-3-(1-piperidyl-sulfonyl)-benzoesäuremethylester (Schmp. 110 - 112°C) und Guanidin in Methanol als Lösungsmittel.
Weißes Kristallpulver, Schmp. 232-234°C.

### Beispiel 9:

### 3-N-Benzylsulfamoyl-4-chlorbenzoyl-guanidin-hydrochlorid

erhält man analog der in Beispiel 1 angegebenen Vorschrift aus 3-N-Benzylsulfamoyl-4-chlorbenzoesäuremethylester (Schmp. 93-95°C) und Guanidin in Methanol als Reaktionsmedium.
Weißes Kristallpulver, Schmp. 224-225°C.

### Beispiel 10

### 4-Sulfamoylbenzoyl-guanidin

erhält man analog der in Beispiel 1 angegebenen Vorschrift aus 4-Sulfamoylbenzoesäure-pentafluorphenolester, dargestellt aus 4-Sulfamoylbenzoylchlorid und Pentafluorphenol in Gegenwart von 1 Mol Pyridin, mit Guanidin in Methanol als Reaktionsmedium.
Weißes Kristallpulver, Schmp. 200°C (Zers.).

### Beispiel 11

### 4-Sulfamoylbenzoyl-guanidin-hydrochlorid

erhält man durch Behandeln der in Beispiel 10 hergestellten Verbindung mit etherischer HCl in Essigsäureethylester.
Farbloses Kristallpulver, Schmp. 282°C.

### Beispiel 12

### 4-N,N-Diethylsulfamoylbenzoyl-guanidin-hydrochlorid

erhält man analog der in Beispiel 1 angegebenen Vorschrift aus 4-N,N-Diethylsulfamoylbenzoesäure-pentafluorphenolester, dargestellt aus 4-N,N-Diethylsulfamoylbenzoesäure, Pentafluorphenol, Thionylchlorid und Pyridin (Schmp. 113-114°C), und Guanidin in Methanol als Lösungsmittel und analoger Aufarbeitung.
Weißes Kristallpulver, Schmp. 216-218°C.

### Beispiel 13

### 4-(1-Piperidylsulfonyl)-benzoylguanidin-hydrochlorid

erhält man analog der in Beispiel 1 angegebenen Vorschrift aus 4-(1-Piperidylsulfonyl)-benzoesäuremethylester und Guanidin in Methanol als Reaktionsmedium.
Weiße Kristalle, Schmp. 281°C.

### Beispiel 14

### 4-Methylthiobenzoyl-guanidin-hydrochlorid

erhält man analog der in Beispiel 1 angegebenen Vorschrift aus 4-Methylthiobenzoesäuremethylester und Guanidin in Methanol als Lösungsmittel.
Weißes Kristallpulver, Schmp. 255-259°C.

### Beispiel 15

### 4-Phenylthio-3-sulfamoylbenzoyl-guanidin-hydrochlorid

a) erhält man analog der in Beispiel 1 angegebenen Vorschrift aus 4-Phenylthio-3-sulfamoylbenzoesäure-methylester und Guanidin in Methanol als Lösungsmittel.
   Weißes Kristallpulver, Schmp. 275-283°C.
b) Den verwendeten 4-Phenylthio-3-sulfamoylbenzoesäuremethylester erhält man durch Umsetzung von 4,4 g 4-Fluor-3-sulfamoylbenzoesäuremethylester (Schmp. 127-129°C), dargestellt aus 4-Fluor-3- sulfamoylbenzoesäure und Thionylchlorid und anschließende Methanolbehandlung, mit 2,2 g Thiophenol und 1,38 g wasserfreiem und gemahlenem K₂CO₃ in 20 ml wasserfreiem Dimethylformamid über 2 Stunden bei 80°C, anschließendes Ausfällen durch Zugabe von ca. 100 ml Wasser.
   Weiße Kristalle aus Methanol, Schmp. 154-157°C.

### Beispiel 16

### 3-Methylsulfonyl-4-phenylthiobenzoyl-guanidin-hydrochlorid

a) erhält man analog der in Beispiel 1 angegebenen Vorschrift aus 3-Methylsulfonyl-4-phenylthiobenzoesäure-methylester und Guanidin in Methanol als Reaktionsmedium und analoger Aufarbeitung.
   Farblose Kristalle, Schmp. 279-281°C.
b) Der verwendete 3-Methylsulfonyl-4-phenylthiobenzoesäuremethylester wird aus 4-Chlor-3-methylsulfonylbenzoesäure-methylester nach der in Vorschrift 15b) angegebenen Verfahrensweise hergestellt.
   Weiße Kristalle, Schmp. 155-156°C.

### Beispiel 17:

### 4-Phenoxy-3-sulfamoylbenzoyl-guanidin

a) erhält man analog der in Beispiel 1 angegebenen Vorschrift (ohne HCl-Behandlung) aus 4-Phenoxy-3-sulfamoylbenzoesäuremethylester und Guanidin in Methanol.
   Weißes Kristallpulver, Schmp. 178-181°C.
b) Den verwendeten 4-Phenoxy-3-sulfamoylbenzoesäure-methylester erhält man analog der in 15 b) angegebenen Vorschrift durch Umsetzung von 4-Fluor-3-sulfamoylbenzoesäure mit Phenol in DMF und in Gegenwart von K₂CO₃ unter Rühren über 6 Stunden bei 80°C.
   Weißer kristalliner Feststoff, Schmp. 148-151°C.

### Beispiel 18

### 3-Methylsulfonyl-4-N-morpholinobenzoyl-guanidin

erhält man analog der in Beispiel 4 f) angegebenen Vorschrift durch Umsetzung von 3-Methylsulfonyl-4-N-morpholino-benzoylchlorid mit Guanidin in wasserfreiem Tetrahydrofuran.
Weißes Kristallpulver, Schmp. 260°C (Zers.)

Das 3-Methylsulfonyl-4-N-morpholino-benzoylchlorid erhält man über mehrere Stufen
a) ausgehend von der 4-Chlor-3-methylsulfonylbenzoesäure, die bei 110°C für 5 Stunden in der doppelten Gewichtsmenge Morpholin gerührt wird und nach Aufnehmen des Reaktionsgemisches in Wasser, woraus nach Acidifizieren mit HCl die 3-Methylsulfonyl-4-N-morpholinobenzoesäure (Schmp. 252-255°C aus Methanol) erhalten wird.
b) 5 g der unter a) genannten 4-Chlor-3-methylsulfonylbenzoesäure werden in 50 ml Thionylchlorid über 2 Stunden am Rückflußkühler gekocht und nach Abdestillieren des flüssigen Bestandteile das gewünschte Benzoylchlorid-Derivat in Diisopropylether/Petrolether-Mischung zur Kristallisation gebracht. Schmp. 98-102°C.

### Beispiel 19

### 3-Methylsulfonyl-4-N-pyrrolidinobenzoyl-guanidin

erhält man analog der in Beispiel 4 f) angegebenen Vorschrift durch Umsetzung von 5 g 3-Methylsulfonyl-4-N-pyrrolidinobenzoyl-chlorid mit 3,4 g Guanidin in wasserfreiem Tetrahydrofuran bei Zimmertemperatur, Eingießen in 200 ml Wasser und Extraktion mit Essigsäureethylester. Nach Abdestillieren des Extraktionsmittels bringt man den Feststoff unter Methanol zur Kristallisation, löst den Feststoff in Essigsäureethylester und reinigt durch Säulenchromatografie an Kieselgel (Laufmittel Essigsäureethylester). Nach Verdampfen des Elutionsmittels kristallisiert man unter Diisopropylether.
Farblose kristalline Verbindung, Schmp. 128-135°C (Zers.).

Das verwendete 3-Methylsulfonyl-4-N-pyrrolidinobenzoylchlorid erhält man über mehrere Stufen ausgehend von der
a) 4-Chlor-3-methylsulfonyl-benzoesäure analog der in Beispiel 18 a) angegebenen Vorschrift durch 5 stündiges Erhitzen auf 100°C in Pyrrolidin und analoger Isolierung der entstandenen 3-Methyl-sulfonyl-4-pyrrolidinylbenzoesäure: Farblose Kristalle aus Ethanol, Schmp. 228-230°C.
   b) Die unter a) hergestellte Benzoesäure wird analog Beispiel 18b) in das gewünschte 3-Methylsulfonyl-4-N-pyrrolidinobenzoylchlorid (Schmp. 137-139°C) umgewandelt.

### Beispiel 20

### 4-N-Morpholino-3-sulfamoylbenzoyl-guanidin

erhält man analog der in Beispiel 1 angegebenen Vorschrift aus dem 4-N-Morpholino-3-sulfamoylbenzoesäuremethylester und Guanidin durch 4 stündiges Kochen in Methanol, Isolierung der Titelverbindung nach der Extraktion mit Essigsäureethylester und säulenchromatographische Reinigung an Kieselgel mit Essigester als Elutionsmittel (ohne anschließende Überführung mit HCl in das Hydrochlorid).
Weißes Kristallpulver, Schmp. 239-241°C.

Den als Ausgangsmaterial verwendeten 4-N- Morpholino-3-sulfamoylbenzoesäuremethylester erhält man durch Erhitzen von 2 g 4-Fluor-3-sulfamoylbenzoesäuremethylester mit 1,5 g Morpholin in 8 ml Dimethylacetamid über 5 Stunden auf 100°C, anschließende Ausfällung der Substanz durch Zugabe von Wasser und Kristallisation des amorphen Esterniederschlages unter Wasser.
Schmp. 61-64°C.

### Beispiel 21

### 3-Methylsulfonyl-4-(1-methyl-4-piperazino)-benzoyl-guanidin

erhält man analog der in Beispiel 1 angegebenen Vorschrift durch Umsetzung von 1 g 3-Methylsulfonyl-4-(1-methyl-4-piperazino)- benzoesäure-methylester und 1 g Guanidin in 25 ml Methanol als Reaktionsmedium über 6 Stunden bei 60°C. Die Verbindung fällt beim Stehenlassen über Nacht aus und wird abfiltriert.
Blaßgelber kristalliner Feststoff, Schmp. 248-250°C.

Den als Ausgangsmaterial verwendeten 3-Methylsulfonyl-4-(1-methyl-4-piperazino)-benzoesäuremethylester erhält man über mehrere Reaktionsstufen ausgehend von
a) 4,7 g 4-Chlor-3-methylsulfonylbenzoesäure und deren Umsetzung mit 5,2 g N-Methylpiperazin in 15 ml Dimethylacetamid bei 120°C über 5 Stunden, Abdestillieren des Lösungsmittels, Aufnehmen des Rückstandes in Wasser, Ansäuern mit 2N Salzsäure und Abfiltrieren des Niederschlages. Kristalliner Feststoff, Schmp. > 300°C.
b) Rückfluß des unter a) hergestellten Benzoesäurederivates in Thionylchlorid, dessen Destillation und Umsetzung des Rückstands mit Methanol und Triethylamin. Nach erneutem Abdestillieren des Lösungsmittels und Behandeln des Rückstandes mit Wasser kommt es zur Abscheidung gewünschten Methylesters, der unter Diisopropylether zur Kristallisation gebracht wird.
   Weißer kristalliner Feststoff, Schmp. 138-145°C.

### Beispiel 22

### 4-N-Benzyl-N-methylamino-3-methylsulfonylbenzoyl-guanidin-hydrochlorid

erhält man analog zu der in Beispiel 4 f) angegebenenen Vorschrift durch Umsetzung von 4,3 g 4-N-Benzyl-N-methylamino-3-methylsulfonyl-benzoylchlorid mit 5,2 g Guanidin in 30 ml abs. Tetrahydrofuran bei 5 bis 10°C. Vor der Überführung mit methanolischem Chlorwasserstoff wird das freie Benzoylguanidin der Titelverbindung durch Säulenchromatographie an Kieselgel mit einem Gemisch aus Essigsäureethylester (10Vol) + Cyclohexan (5Vol) + Chloroform (5Vol) + Methanol (5Vol) + NH₃-aq. (1Vol) als Elutionsmittel gereinigt.
Farbloses Kristallpulver, Schmp. 234-240°C.

Das beschriebene 4-N-Benzyl-N-methylamino-3-methylsulfonyl-benzoylchlorid (ölig amorphes Produkt, das ohne weitere Reinigungsschritte verwendet wird) erhält man über mehrere Stufen ausgehend von der 4-Chlor-3-methylsulfonylbenzoesäure, die in Analogie zu Beispiel 21, a) mit N-Benzyl-N-methylamin zur 4-N-Benzyl-N-methylamino-3- methylsulfonylbenzoesäure (Schmp. 196-202°C) reagiert, und anschließende Reaktion mit Thionylchlorid.

### Beispiel 23

### 4-Benzylamino-3-methylsulfonylbenzoyl-guanidin-acetat

erhält man analog zu der in Beispiel 4 f) beschriebenen Methode durch Umsetzung von 4-Benzyl-3-methylsulfonyl-benzoylchlorid mit Guanidin in wasserfreiem Tetrahydrofuran. Die Aufarbeitung erfolgt durch Acidifizieren mit Essigsäure auf pH 4; nach dem teilweisen Abdestillieren des Lösungsmittels, insbesondere des THF-Anteils, scheidet sich das 4-Benzylamino-3-methylsulfonylbenzoyl-guanidin-acetat kristallin ab.
Hellgelber kristalliner Feststoff mit 2 Schmelzpunkten:
Schmp.₁ 163-168°C, Schmp.₂ 230-232°C.

Die als Vorstufe verwendete 4-Benzylamino-3-methylsulfonyl-benzoesäure (Schmp. 230-235°C) erhält man in Analogie zu der in Beispiel 21 beschriebenen Reaktionsdurchführung aus 4-Chlor-3-methyl-sulfonylbenzoesäure und Benzylamin und in obiger Analogie mit Thionylchlorid das gewünschte 4-Benzyl-3-methylsulfonyl- benzoylchlorid (Schmp. 100-104°C).

Analog der in Beispiel 4 f) angegebenen Vorschrift erhält man aus den entsprechenden Benzoylchloriden der Formel II und Guanidin in wasserfreiem Tetrahydrofuran die nachfolgenden Verbindungen der Formel I bzw. deren Salze:

### Beispiel 24:

3-(3-Methyl-1-butylsulfonyl-4-N-piperidino-benzoylguanidin-hydrochlorid (Schmp. 155°C/Zers.)
mit 2-Chlor-5-carboxybenzolsulfinsäure-dinatriumsalz als Ausgangsstufe über 4-Chlor-3-(3-methyl-1-butylsulfonyl)-benzoesäure-3-methyl-1-butylester (amorphe Verbindung),
4-Chlor-3-(3-methyl-1-butylsulfonyl)-benzoesäure (Schmp. 140- 144°C),
3-(3-Methyl-1-butylsulfonyl)-4-N-piperidino-benzoesäure
(Schmp. 161-165°C)
und 3-(3-Methyl-1-butylsulfonyl)-4-N-piperidino-benzoylchlorid (Öl).

### Beispiel 25

### 3-(1-Butylsulfonyl)-4-N-piperidinobenzoyl-guanidin-dihydrochlorid (Schmp. 184°C/Zers.)

mit 2-Chlor-5-carboxybenzolsulfinsäure-dinatriumsalz als Ausgangsstufe über 3-(1-Butylsulfonyl)-4-chlorbenzoesäure-1-butyl- ester (Öl),
3-(1-Butylsulfonyl)-4-chlorbenzoesäure (Schmp. 142-146°C),
3-(1-Butylsulfonyl)-4-N-piperidinobenzoesäure (Schmp. 156°C)
und 3-(1-Butylsulfonyl)-4-N-piperidinobenzoylchlorid (Öl).

### Beispiel 26

### 4-N-(1-Hexylamino)-3-methylsulfonylbenzoyl-guanidin

Zu einer Lösung von 2,99 g 4-N-(1-Hexylamino)-3-methylsulfonyl- benzoesäure in 40 ml wasserfreiem Tetrahydrofuran gibt man 1,78 g Carbonyldiimidazol, rührt 2 Stunden bei Zimmertemperatur und 1 Stunde bei 45°C und versetzt die Mischung anschließend bei 20°C mit 4,13 g Guanidin. Man rührt 12 Stunden bei Zimmertemperatur, destilliert das Lösungsmittel ab, stellt mit 2N HCl auf pH 6-7, rührt eine Stunde im Eisbad, filtriert den Feststoff ab und löst diesen nach dem Waschen mit Wasser aus Ethanol um.
Farbloser Feststoff, Schmp. 135-145°C.

Vorstufen von Beispiel 26 ausgehend von 4-Chlor-3-methylsulfonyl- benzoesäure über 4-N-(1-Hexylamino)-3-methylsulfonylbenzoesäure (farblose Kristalle, Schmp. 169°C).

### Beispiel 27

### 3-N-Ethyl-N-isopropylsulfamoyl-4-(1-piperidino)-benzoylguanidin

erhält man analog der in Beispiel 26 angegebenen Vorschrift durch Umsetzung von 3-N-Ethyl-N-isopropylsulfamoyl-4-(1-piperidino)-benzoesäure, Carbonyldiimidazol und Guanidin.
Farblose Kristalle, Schmp. 212°C.

Vorstufen von Beispiel 27 ausgehend von 3-Chlorsulfonyl-4-fluor-benzoesäure über 3-N-Ethyl-N-isopropylsulfamoyl-4-fluorbenzoesäure (Schmp. 108-115°C), 3-N-Ethyl-N-isopropylsulfamoylbenzoesäure (Schmp. 138-140°C).

### Beispiel 28

### 4-Amino-3-methylsulfonylbenzoyl-guanidin-hydrochlorid

erhält man analog der in Beispiel 1 angegebenen Vorschrift durch Umsetzung von 4-Amino-3-methylsulfonylbenzoesäuremethylester und Guanidin in Methanol.
Weißes Kristallpulver, Schmp. 245-248°C.

Vorstufen von Beispiel 28 ausgehend von 4-Chlor-3-methylsulfonyl-benzoesäure über 4-N-Hydrazino-3-methylsulfonylbenzoesäure (Schmp. 243-245°C) und dessen hydrogenolytische N-N-Spaltung mit Raney-Nickel-Katalysator in Methanol/NH₃-aq. unter Normaldruck bei Zimmertemperatur zur 4-Amino-3-methylsulfonylbenzoesäure (Schmp. 258- 262°C) und nachfolgende Umsetzung mit Thionylchlorid und Methanol zu 4-Amino-3-methylsulfonylbenzoesäuremethylester (Schmp. 146°C).

### Beispiel 29

### 1,1-Dimethyl-3-(3-methylsulfonyl-4-N-piperidinobenzoyl)-guanidin

3,6 g 3,5-Dimethylpyrazol-1-carbonsäure-N-(3-methylsulfonyl-4-(1-piperidino)-benzoyl)-carbonsäureamidin werden in 30 ml 40%iger wäßriger Dimethylaminlösung im Autoklaven über 4 Stunden auf 80°C erwärmt, der kristalline Niederschlag abfiltriert und aus Ethanol umkristallisiert.
Farblose Kristalle, Schmp. 207°C.

Vorstufen von Beispiel 29 ausgehend von einer Lösung aus 2,92 g 3- Methylsulfonyl-4-N-piperidino-benzoylchlorid in 10 ml Essigester und deren Zutropfen zu einer Lösung von 3,5-Dimethylpyrazol-1-carbonsäureamidin Nitrat (97%, Aldrich) in 12,8 ml 10%iger NaOH bei 10-15°C, 2 stündiges Rühren bei Zimmeremperatur, Abdestillieren des Lösungsmittels und Umkristallisieren des Rückstandes aus Essigsäureethylester (Farbloser kristalliner Feststoff, Schmp. 201-202°C).

### Beispiel 30

### 1-Methyl-3-(3-methylsulfonyl-4-N-piperidino-benzoyl)-guanidin

erhält man analog der in Beispiel 29 angegebenen Vorschrift durch Umsetzung mit 40%iger wäßriger Methylaminlösung im Autoklaven.
Farbloser kristalliner Feststoff, Schmp. 202-203°C (aus Ethanol).

### Beispiel 31

### 4-Chlor-3-(3-phenyl-1-propylsulfonyl)-benzoyl-guanidin-hydrochlorid

erhält man analog der in Beispiel 26 beschriebenen Vorschrift durch Umsetzung von 4-Chlor-3-(3-phenyl-1-propylsulfonyl)-benzoesäure mit Guanidin und Carbonyldiimidazol.
Farblose Kristalle, Schmp. 178-182°C (Zers.).

Vorstufen von Beispiel 31 ausgehend von 2-Chlor-5-carboxybenzol-sulfinsäure über 4-Chlor-3-(3-phenyl-1-propylsulfonyl)-benzoesäure-(3-phenyl-1-propyl)-ester (viskoses Öl) und 4-Chlor-3-(3-phenyl-1-propylsulfonyl)-benzoesäure (farblose Kristalle, Schmp. 126-130°C).

### Beispiel 32

### 4-(2,3-Dihydro-1-indolyl)-3-methylsulfonylbenzoyl-guanidin

erhält man analog zu der in Beispiel 26 angegebenen Vorschrift aus 4-(2,3-Dihydro-1-indolyl)-3-methylsulfonylbenzoesäure, Carbonyldiimidazol und Guanidin (Schmp. 136°C/Zers.).

Vorstufen von Beispiel 32 ausgehend von 4-Chlor-3-methylsulfonyl-benzoesäure über 4-(2,3-Dihydro-1-indolyl)-3-methylsulfonyl-benzoesäure (Schmp. 158°C).

### Beispiel 33:

### 3-(3-Phenyl-1-propylsulfonyl)-4-(1-piperidino)-benzoyl-guanidin-dihydrochlorid

erhält man analog der in Beispiel 26 angegebenen Vorschrift durch Umsetzung von 3-(3-Phenyl-1-propylsulfonyl)-4-(1-piperidino)-benzoesäure, Carbonyldiimidazol und Guanidin.
Farbloser Feststoff, Schmp. 160°C (Zers.).

Vorstufen: Ausgehend von 4-Chlor-3-(3-phenyl-1-propylsulfonyl)- benzoesäure (siehe Bsp. 31) über 3-(3-Phenyl-1-propylsulfonyl)-4-(1- piperidino)-benzoesäure (Schmp. 170°C).

### Beispiel 34

### 4-(N-2,4-Dichlorbenzyl-N-methylamino)-3-methylsulfonylbenzoyl-guanidin-hydrochlorid

erhält man analog der in Beispiel 1 angegebenen Vorschrift durch Umsetzung von 4-(N-2,4-Dichlorbenzyl-N-methylamino)-3-methylsulfonyl-benzoesäuremethylester und Guanidin in Methanol als Lösungsmittel. (Schmp: 180°C)

### Beispiel 35

### 4-(1-Piperidino)-3-sulfamoylbenzoyl-guanidin-hydrochlorid

erhält man analog der in Beispiel 26 angegebenen Vorschrift aus 4-(1-Piperidino)-3-sulfamoyl-benzoesäure, Carbonyldiimidazol und Guanidin.
Farbloser kristalliner Feststoff, Schmp. 236°C (Zers.).

Vorstufen ausgehend von 4-Fluor-3-sulfamoylbenzoesäure über 4-(1-Piperidino)-3-sulfamoylbenzoesäure (Schmp. 246-247°C).

Analog der in Beispiel 26 angegebenen Vorschrift erhält man durch Umsetzung eines Benzoesäure-Derivates der Formel II mit X=OH, dessen Aktivierung mit Carbonyldiimidazol und anschließende Umsetzung mit Guanidin die nachfolgend aufgeführten Verbindungen der Formel I:

### Beispiel 36:

### 4-(3-Methoxy-1-propylamino)-3-methylsulfonylbenzoyl-guanidin-hydrochlorid, Farblose Kristalle, Schmp. 167-169°C

(Vorstufen ausgehend von 4-Chlor-3-methylsulfonyl-benzoesäure über 4-(3-Methoxy-1-propylamino)-3-methylsulfonylbenzoesäure (Schmp. 190°C/aus Ethanol).

### Beispiel 37:

### 4-(3-Phenyl-1-propylamino)-3-methylsulfonylbenzoyl-guanidin Weißes Kristallpulver, Schmp. 177-178°C.

(Vorstufen ausgehend von 4-Chlor-3-methylsulfonylbenzoesäure über 4-(3-Phenyl-1-propylamino)-3-methylsulfonylbenzoesäure (Schmp. 172°C).

### Beispiel 38:

### 4-Isobutylamino-3-methylsulfonylbenzoyl-guanidin-hydrochlorid Weißes Kristallpulver, Schmp. 163-166°C

(Vorstufen ausgehend von 4-Chlor-3-methylsulfonylbenzoesäure über 4-Isopropylamino-3-methylsulfonylbenzoesäure, Schmp. 180°C)

### Beispiel 39:

### 3-(3-Methoxy-1-propylsulfamoyl)-4-(1-piperidino)-benzoyl-guanidin-hydrochlorid Weißes Kristallpulver, Schmp. 209-211°C.

(Vorstufen ausgehend von 4-Chlor-3-chlorsulfonylbenzoesäure über 4-Chlor-3-(3-methoxy-1-propylsulfamoyl)-benzoesäure (Schmp. 149-150°C) und 3-(3-Methoxy-1-propylsulfamoyl)-4-(1-piperidino)-benzoesäure (Schmp. 138-140°C).

### Beispiel 40

### 3-Isopropylsulfamoyl-4-(1-piperidino)-benzoyl-guanidin-hydrochlorid Weißes Kristallpulver, Schmp. 209-211°C.

Vorstufen ausgehend von 4-Chlor-3-isopropylsulfamoylbenzoesäure über 3-Isopropylsulfamoyl-4-(1-piperidino)-benzoesäure (Schmp. 186- 188°C).

### Beispiel 41

### 3-(1-Propylsulfamoyl)-4-(1-piperidino)-benzoyl-guanidin-hydrochlorid Weißes Kristallpulver, Schmp. 176°C (Zers)

Vorstufen: Ausgehend von 4-Chlor-3-(1-propylsulfamoyl)-benzoesäure über 3-(1-Propylsulfamoyl)-4-(1-piperidino)-benzoesäure (Schmp. 138-139°C).

### Beispiel 42

### 3-(3,5-Dimethyl-1-piperidino-sulfonyl)-4-(1-piperidino)-benzoyl-guanidin Weißes Kristallpulver, Schmp. 202-204°C.

Vorstufen: Ausgehend von 3-Chlorsulfonyl-4-fluorbenzoesäure über 3-(3,5-Dimethyl-1-piperidinosulfonyl)-4-fluor-benzoesäure (Schmp. 207°C), 3-(3,5-Dimethyl-1-piperidinosulfonyl)-4-(1-piperidino)-benzoesäure (Schmp. 198°C).

### Beispiel 43

### 3-Methylsulfonyl-4-(N,N-di-1-propylamino)-benzoyl-guanidin-hydrochlorid Weißes, Kristallpulver, Schmp. 157-9°C

Vorstufen: Ausgehend von 3-Methylsulfonyl-4-chlorbenzoesäure über 3-Methylsulfonyl-4-(N,N-di-1-propylamino)-benzoesäure (Schmp. 122- 124°C).

### Beispiel 44

### 4-Cyclopentylamino-3-methylsulfonylbenzoyl-guanidin Weißes Kristallpulver, Schmp. 238-40°C.

Vorstufen:Ausgehend von 4-Chlor-3-methylsulfonylbenzoesäure über 4-Cyclopentylamino-3-methylsulfonylbenzoesäure (Schmp. 219-222°C).

### Beispiel 45

### 4-Ethylsulfonyl-3-N-pyrrolidinobenzoyl-guanidin-hydrochlorid

Analog Beispiel 2 aus 4-Ethylsulfonyl-3-N-pyrrolidinobenzoesäuremethylester und Guanidin
Schmp.: 180-182°C

### Beispiel 46

### 4-Phenylsulfonyl-3-N-pyrrolidinobenzoyl-guanidin-hydrochlorid

Analog Beispiel 2 aus 4-Phenylsulfonyl-3-N-pyrrolidinobenzoesäuremethylester und Guanidin.
Schmp.: 226-228°C

### Beispiel 47

### 4-Cyclohexylsulfonyl-3-N-pyrrolidinobenzoyl-guanidin

Analog Beispiel 2 aus 6-Cyclohexylsulfonyl-3-N-pyrrolidinobenzoesäuremethylester und Guanidin. Das zunächst erhaltene Hydrochlorid der Titelverbindung wird in H₂O gelöst und durch Zugabe von 2N Natronlauge als freie Base gefällt und abgesaugt.
Schmp.: 234-36°C

### Beispiel 48

### 4-Methylsulfonyl-3-N-pyrrolidinobenzoyl-guanidin

Analog Beispiel 2 aus 4-Methylsulfonyl-3-N-pyrrolidino-benzoesäuremethylester und Guanidin
Schmp.: 165-168°C

Herstellung der Vorprodukte zu den Beispielen 45-48:
Für die Herstellung der entsprechenden Benzoesäuremethylester für die Beispiele 45-48 verwendet man 4-Chlor-3-nitrobenzoesäuremethylester als Ausgangsmaterial. Umsatz mit Ethylmercaptan (45), Thiophenol (46), Cyclohexylsulfid (47) oder Methylmercaptan (48) führt unter Standardbedingungen wie K₂CO₃ in DMF zu den entsprechenden 4-Thio-3-nitrobenzoesäuremethylestern, die mit H₂O₂ in Eisessig zu den entsprechenden 4-Sulfonyl-3-nitrobenzoesäureestern oxidiert werden. Nach Reduktion der 3-Nitrogruppe wird mit Bernsteinsäureanhydrid und Reduktion mit NaBH₄/BF₃·Et₂O der Pyrrolidinrest eingeführt:
10 g 3-Amino-4-methylsulfonylbenzoesäuremethylester werden mit 8,7 g Bernsteinsäureanhydrid 5 Stunden in der Schmelze bei 160°C gerührt. Der feste Rückstand wird in Methanol gelöst und in Wasser eingerührt. Der ausgefallene Niederschlag wird abgesaugt.
Schmp.: 218-220°C
12,2 g des hiermit hergestellten 4-Methylsulfonyl-3-N-(2,5-Dioxopyrrolidinobenzoesäuremethylesters löst man in 100 ml Diglyme und kühlt nach Zugabe von 14,5 ml BF₃-Etherat auf 2°C. Anschließend addiert man 4,4 g NaBH₄ und rührt die Mischung 5 Stunden bei dieser Temperatur. Anschließend wird der Ansatz auf Eis/Wasser gegossen und der ausgefallene 4-Methylsulfonyl-3-N-pyrrolidinobenzoesäuremethylester (Schmp.: 96-98°C) abgesaugt.
Hierzu analog erhält man die entsprechenden Benzoesäureester für die Beispiele 45,46 und 47.

### Beispiel 49

### 4-Cyclohexylthio-3-methylsulfonylbenzoyl-guanidin

Analog Beispiel 1, jedoch mit 4-Cyclohexylthio-3-methylsulfonylbenzoesäuremethylester als Ausgangsmaterial.
Schmp.: 201-204°C

### Pharmakologische Daten

### K-Strophanthidin-induzierte ventrikuläre Tachykardie am Hund

### Methode:

Intravenöse Infusion von 3 »g/kg·min Ouabain führt an Pentobarbitonnarkotisierten Beagles zu einer anhaltenden ventrikulären Tachykardie (VT) innerhalb von 40-60 Minuten. Sobald VT einsetzt, wird die Ouabaininfusion abgebrochen, die VT bleibt jedoch für annähernd 2 Stunden weiter bestehen und wird durch fortwährend EKG-Kontrolle verfolgt. Die zu prüfende Substanz wird 10 min nach Beendigung der Ouabain-Infusion i.v. appliziert.

### Ergebnisse:

In 4 von 5 Hunden führten 10 mg/kg der Verbindung aus Beispiel 34 zu einer Normalisierung des EKGs für 6-11 Minuten.

## Patentansprüche (Patentansprüche für folgende(n) Vertragsstaat(en): AT, BE, CH, DE, DK, FR, GB, IT, LI, LU, NL, SE)

1. Benzoylguanidine der Formel I 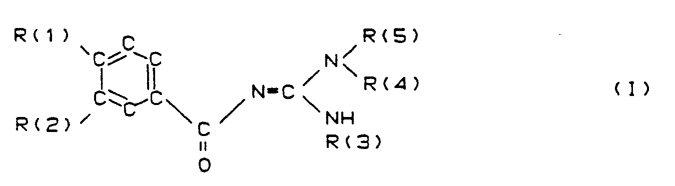 worin R(1) oder R(2) R(6)-S(O)ₙ- oder 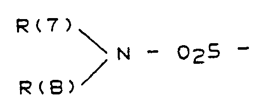 bedeuten
und der jeweils andere Substituent R(1) oder R(2)
H, F, Cl, Br, J, C₁-C₄-Alkyl, C₁-C₄-Alkoxy, Phenoxy, das unsubstituiert ist oder einen bis drei Substituenten aus den Gruppen Fluor, Chlor, Methyl oder Methoxy trägt,
R(6)-S(O)ₙ oder 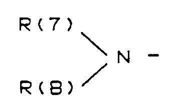 bedeutet,
und worin R⁶ für C₁-C₆-Alkyl, C₅-C₇-Cycloalkyl, Cyclopentylmethyl, Cyclohexylmethyl oder für Phenyl steht, das unsubstituiert ist oder einen bis drei Substituenten aus den Gruppen Fluor, Chlor, Methyl oder Methoxy trägt,
und worin R(7) und R(8) gleich oder verschieden sind und in der Bedeutung von H, C₁-C₆-Alkyl stehen oder R(7) für Phenyl-(CH₂)ₘ- mit m=1-4 oder Phenyl steht, das unsubstituiert ist oder einen bis zwei Substituenten aus den Gruppen Fluor, Chlor, Methyl oder Methoxy trägt,
und worin R(7) und R(8) auch gemeinsam eine geradkettige oder verzweigte C₄-C₇-Kette sein können, wobei die Kette zusätzlich durch O, S oder NR(9) unterbrochen sein kann und R(9) für H oder Methyl steht
und worin R(7) und R(8) gemeinsam mit dem Stickstoffatom, an das sie gebunden sind, ein Dihydroindol, Tetrahydrochinolin oder Tetrahydroisochinolin-System bedeuten können,
und R(3), R(4) und R(5) Wasserstoff, C₁-C₂-Alkyl bedeuten, wobei R(3) und R(4) gemeinsam eine (C₂-C₄)-Alkylenkette und R(4) und R(5) gemeinsam eine (C₄-C₇)-Alkylenkette bilden können,
und n für Null, 1 oder 2 steht,
sowie deren pharmazeutisch verträgliche Salze.

2. Verbindung I nach Anspruch 1, worin bedeuten:
R(1) Fluor, Chlor, NR(7)R(8), R(6)-S(O)ₙ- oder Phenoxy bedeutet,
R(2) für R(6)-S(O)ₙ- und R(7)R(8)N-SO₂- steht
und worin n Null, 1, 2 ist
und R(3), R(4) und R(5) Wasserstoff,
und worin R(6), R(7) und R(8) die oben angegebene Bedeutung haben und deren pharmakologisch verträgliche Salze.

3. Verbindung I nach Anspruch 1, worin bedeuten:
R(1) R(7)R(8)N- oder R(6)-S(O)ₙ-, worin R(6) Alkyl mit 1-3 C-Atomen oder Phenyl, n=Null
und R(7) und R(8) Wasserstoff, Alkyl mit 1-4 C-Atomen oder R(7) und R(8) gemeinsam eine (C₄-C₇)-Methylenkette bilden und
R(2) für CH₃-SO₂- oder H₂N-SO₂- steht, R(3), R(4) und R(5) Wasserstoff, und deren pharmakologisch verträgliche Salze.

4. Verbindung I nach Anspruch 1, dadurch gekennzeichnet, daß sie
3-Methylsulfonyl-4-(1-piperidinyl)-benzoyl-guanidin, 4-Phenylthio-3-sulfamoylbenzoyl-guanidin oder 3-Methylsulfonyl-4-phenylthiobenzoyl-guanidin oder ein pharmakologisch verträgliches Salz ist.

5. Verfahren zur Herstellung einer Verbindung I nach Anspruch 1,
dadurch gekennzeichnet, daß man Verbindungen der Formel II 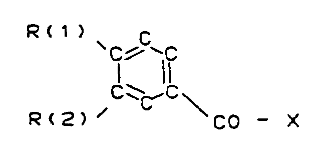 mit einem Guanidinderivat der Formel III 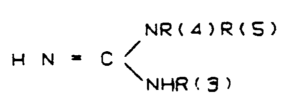 umsetzt, worin R(1) bis R(5) die in Anspruch 1 angegebene Bedeutung besitzen und X für eine leicht nucleophil substituierbare leaving group steht.

6. Verwendung einer Verbindung I nach Anspruch 1 zur Herstellung eines Medikaments zur Behandlung von Arrhythmien.

7. Verwendung einer Verbindung I nach Anspruch 1 zur Herstellung eines Medikaments zur Behandlung oder Prophylaxe von ischämischen Zuständen des Herzens.

8. Arzneimittel, welches eine wirksame Menge einer Verbindung I nach Anspruch 1 enthält.

## Patentansprüche (Patentansprüche für folgende(n) Vertragsstaat(en): ES, GR)

1. Verfahren zur Herstellung eines Benzoylguanidins der Formel I 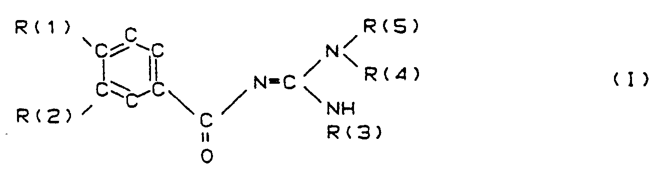 worin R(1) oder R(2) R(6)-S(O)ₙ- oder 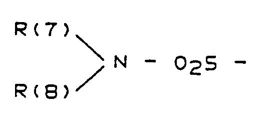 bedeuten
und der jeweils andere Substituent R(1) oder R(2)
H, F, Cl, Br, J, C₁-C₄-Alkyl, C₁-C₄-Alkoxy, Phenoxy, das unsubstituiert ist oder einen bis drei Substituenten aus den Gruppen Fluor, Chlor, Methyl oder Methoxy trägt,
R(6)-S(O)ₙ oder 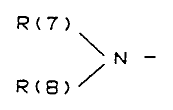 bedeutet,
und worin R⁶ für C₁-C₆-Alkyl, C₅-C₇-Cycloalkyl, Cyclopentylmethyl, Cyclohexylmethyl oder für Phenyl steht, das unsubstituiert ist oder einen bis drei Substituenten aus den Gruppen Fluor, Chlor, Methyl oder Methoxy trägt,
und worin R(7) und R(8) gleich oder verschieden sind und in der Bedeutung von H, C₁-C₆-Alkyl stehen oder R(7) für Phenyl-(CH₂)ₘ- mit m=1-4 oder Phenyl steht, das unsubstituiert ist oder einen bis zwei Substituenten aus den Gruppen Fluor, Chlor, Methyl oder Methoxy trägt,
und worin R(7) und R(8) auch gemeinsam eine geradkettige oder verzweigte C₄-C₇-Kette sein können, wobei die Kette zusätzlich durch O, S oder NR(9) unterbrochen sein kann und R(9) für H oder Methyl steht
und worin R(7) und R(8) gemeinsam mit dem Stickstoffatom, an das sie gebunden sind, ein Dihydroindol, Tetrahydrochinolin oder Tetrahydroisochinolin-System bedeuten können,
und R(3),R(4) und R(5) Wasserstoff, C₁-C₂-Alkyl bedeuten, wobei R(3) und R(4) gemeinsam eine (C₂-C₄)-Alkylenkette und R(4) und R(5) gemeinsam eine (C₄-C₇)-Alkylenkette bilden können,
und n für Null, 1 oder 2 steht,
sowie eines pharmazeutisch verträglichen Salzes,
dadurch gekennzeichnet, daß man eine Verbindung der Formel II 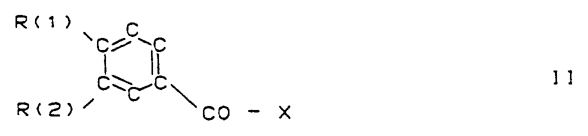 mit einem Guanidinderivat der Formel III 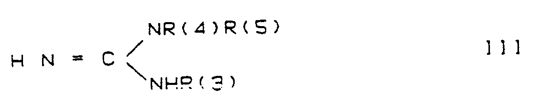 umsetzt, worin R(1) bis R(5) die oben angegebene Bedeutung besitzen und X für eine leicht nucleophil substituierbare leaving group steht.

2. Verfahren nach Anspruch 1, dadurch gekennzeichnet,
daß man Verbindungen II und III einsetzt, in denen die Substituenten folgende Bedeutung haben:
R(1) Fluor, Chlor, NR(7)R(8), R(6)-S(O)ₙ- oder Phenoxy,
R(2) für R(6)-S(O)ₙ- und R(7)R(8)N-SO₂-
worin n Null, 1, 2,
R(3), R(4) und R(5) Wasserstoff,
R(6), R(7) und R(8) wie in Anspruch 1 definiert.

3. Verfahren nach Anspruch 1, dadurch gekennzeichnet, daß man Verbindungen II und III einsetzt, in denen bedeuten:
R(1) R(7)R(8)N- oder R(6)-S(O)ₙ-, worin R(6) Alkyl mit 1-3 C-Atomen oder Phenyl,
n=Null sind,
und R(7) und R(8) Wasserstoff, Alkyl mit 1-4 C-Atomen oder R(7) und R(8) gemeinsam eine (C₄-C₇)-Methylenkette bilden und
R(2) für CH₃-SO₂- oder H₂N-SO₂- und R(3), R(4) und R(5) für Wasserstoff stehen.

4. Verfahren nach Anspruch 1, dadurch gekennzeichnet, daß man die Verbindungen 3-Methylsulfonyl-4-(1-piperidinyl)-benzoyl-guanidin, 4-Phenylthio-3-sulfamoylbenzoyl-guanidin oder 3-Methylsulfonyl-4-phenylthiobenzoyl-guanidin oder deren pharmakologisch verträgliche Salze herstellt.

5. Verfahren zur Herstellung eines Medikaments zur Behandlung von Arrhythmien, dadurch gekennzeichnet, daß man eine wirksame Menge einer Verbindung I nach Anspruch 1 mit den pharmazeutisch üblichen Zusatzstoffen vermischt und in eine geeignete Darreichungsform bringt.

## Claims (Claims for the following Contracting State(s): AT, BE, CH, DE, DK, FR, GB, IT, LI, LU, NL, SE)

1. A benzoylguanidine of the formula I 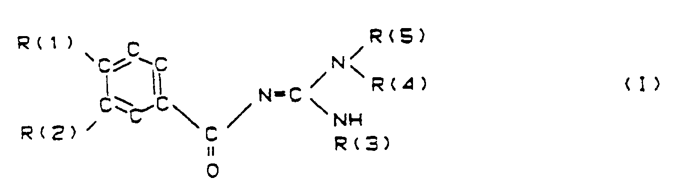 in which R(1) or R(2) is R(6)-S(O)ₙ- or 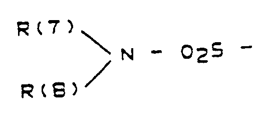 and the other substituent R(1) or R(2) in each case is H, F, Cl, Br, I, C₁-C₄-alkyl, C₁-C₄-alkoxy, phenoxy which is unsubstituted or carries from one to three substituents from the group fluorine, chlorine, methyl or methoxy,
R(6)-S(O)ₙ or 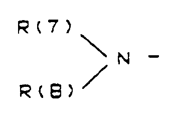 and in which R⁶ is C₁-C₆-alkyl, C₅-C₇-cycloalkyl, cyclopentylmethyl, cyclohexylmethyl or phenyl which is unsubstituted or carries from one to three substituents from the group fluorine, chlorine, methyl or methoxy,
and in which R(7) and R(8) are identical or different and have the meaning of H or C₁-C₆-alkyl, or R(7) is phenyl-(CH₂)ₘ-, with m being 1-4, or phenyl which is unsubstituted or carries from one to two substituents from the group fluorine, chlorine, methyl or methoxy,
and in which R(7) and R(8) can also together be a straight-chain or branched C₄-C₇ chain where the chain can additionally be interrupted by O, S or NR(9) and R(9) is H or methyl,
and in which R(7) and R(8), together with the nitrogen atom to which they are bonded, can be a dihydroindole, tetrahydroquinoline, or tetrahydroisoquinoline system,
and R(3), R(4) and R(5) are hydrogen or C₁-C₂-alkyl, where R(3) and R(4) can together form a (C₂-C₄)-alkylene chain and R(4) and R(5) can together form a (C₄-C₇)-alkylene chain,
and n is zero, 1 or 2,
and the pharmaceutically tolerated salts thereof.

2. A compound I as claimed in claim 1, in which: R(1) is fluorine, chlorine, NR(7)R(8), R(6)-S(O)ₙ- or phenoxy,
R(2) is R(6)-S(O)ₙ- and R(7)R(8)N-SO₂-,
and in which n is zero, 1 or 2,
and R(3), R(4) and R(5) are hydrogen,
and in which R(6), R(7) and R(8) have the abovementioned meaning, and the pharmaceutically tolerated salts thereof.

3. A compound I as claimed in claim 1, in which:
R(1) is R(7)R(8)N- or R(6)-S(O)ₙ-, in which R(6) is alkyl having 1-3 carbon atoms or phenyl,
n is zero,
and R(7) and R(8) are hydrogen or alkyl having 1-4 carbon atoms, or R(7) and R(8) together form a (C₄-C₇)-methylene chain, and
R(2) is CH₃-SO₂- or H₂N-SO₂-, R(3), R(4) and R(5) are hydrogen, and the pharmaceutically tolerated salts thereof.

4. A compound I as claimed in claim 1, which is 3-methylsulfonyl-4-(1-piperidinyl)benzoylguanidine, 4-phenylthio-3-sulfamoylbenzoylguanidine or 3-methylsulfonyl-4-phenylthiobenzoylguanidine or a pharmacologically tolerated salt.

5. A process for preparing a compound I as claimed in claim 1,
wherein compounds of the formula II 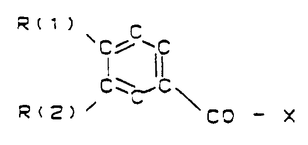 are reacted with a guanidine derivative of the formula III 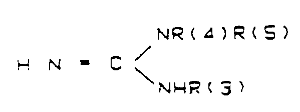 in which R(1) to R(5) have the meaning given in claim 1 and X is a leaving group which can readily be substituted nucleophilically.

6. Use of a compound I as claimed in claim 1 for preparing a medicament for treating arrhythmias.

7. Use of a compound I as claimed in claim 1 for preparing a medicament for the treatment or prophylaxis of ischaemic conditions of the heart.

8. A pharmaceutical which contains an effective quantity of a compound I as claimed in claim 1.

## Claims (Claims for the following Contracting State(s): ES, GR)

1. A process for preparing a benzoylguanidine of the formula I 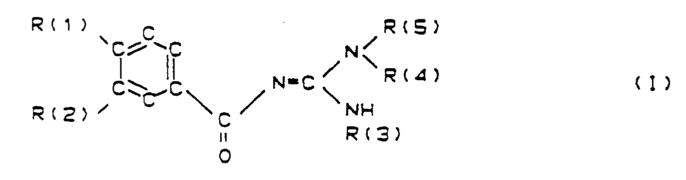 in which R(1) or R(2) is R(6)-S(O)ₙ- or 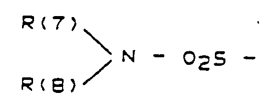 and the other substituent R(1) or R(2) in each case is H, F, Cl, Br, I, C₁-C₄-alkyl, C₁-C₄-alkoxy, phenoxy which is unsubstituted or carries from one to three substituents from the group fluorine, chlorine, methyl or methoxy,
R(6)-S(O)ₙ or 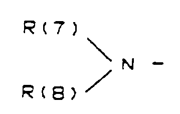 and in which R⁶ is C₁-C₆-alkyl, C₅-C₇-cycloalkyl, cyclopentylmethyl, cyclohexylmethyl or phenyl which is unsubstituted or carries from one to three substituents from the group fluorine, chlorine, methyl or methoxy,
and in which R(7) and R(8) are identical or different and have the meaning of H or C₁-C₆-alkyl, or R(7) is phenyl-(CH₂)ₘ-, with m being 1-4, or phenyl which is unsubstituted or carries from one to two substituents from the group fluorine, chlorine, methyl or methoxy,
and in which R(7) and R(8) can also together be a straight-chain or branched C₄-C₇ chain where the chain can additionally be interrupted by O, S or NR(9) and R(9) is H or methyl,
and in which R(7) and R(8), together with the nitrogen atom to which they are bonded, can be a dihydroindole, tetrahydroquinoline, or tetrahydroisoquinoline system,
and R(3), R(4) and R(5) are hydrogen or C₁-C₂-alkyl, where R(3) and R(4) can together form a (C₂-C₄)-alkylene chain and R(4) and R(5) can together form a (C₄-C₇)-alkylene chain,
and n is zero, 1 or 2,
and a pharmaceutically tolerated salt thereof,
wherein a compound of the formula II 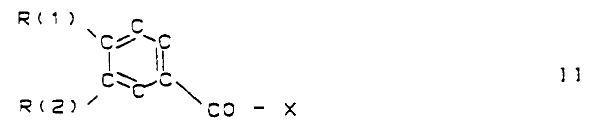 is reacted with a guanidine derivative of the formula III 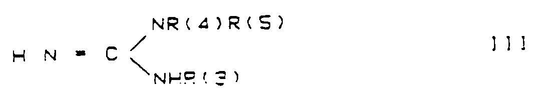 in which R(1) to R(5) have the abovementioned meaning and X is a leaving group which can readily be substituted nucleophilically.

2. The process as claimed in claim 1, wherein compounds II and III are employed in which the substituents have the following meaning:
R(1) is fluorine, chlorine, NR(7)R(8), R(6)-S(O)ₙ- or phenoxy,
R(2) is R(6)-S(O)ₙ- and R(7)R(8)N-SO₂- in
which N is zero, 1 or 2,
R(3), R(4) and R(5) are hydrogen,
R(6), R(7) and R(8) are defined as in claim 1.

3. The process as claimed in claim 1, wherein compounds II and III are employed in which:
R(1) is R(7)R(8)N- or R(6)-S(O)ₙ-, in which R(6) is alkyl having 1-3 carbon atoms or phenyl,
n is zero,
and R(7) and R(8) are hydrogen or alkyl having 1-4 carbon atoms, or R(7) and R(8) together form a (C₄-C₇)-methylene chain, and
R(2) is CH₃-SO₂- or H₂N-SO₂-, and R(3), R(4) and R(5) are hydrogen.

4. The process as claimed in claim 1, wherein the compounds 3-methylsulfonyl-4-(1-piperidinyl)-benzoylguanidine, 4-phenylthio-3-sulfamoylbenzoylguanidine or 3-methylsulfonyl-4-phenylthiobenzoylguanidine, or their pharmacologically tolerated salts, are prepared.

5. A process for preparing a medicament for treating arrhythmias, wherein an effective quantity of a compound I as claimed in claim 1 is mixed with the pharmaceutically customary additives and brought into a suitable form for administration.

## Revendications (Revendications pour l'(les) Etat(s) contractant(s) suivant(s): AT, BE, CH, DE, DK, FR, GB, IT, LI, LU, NL, SE)

1. Benzoylguanidines de formule I 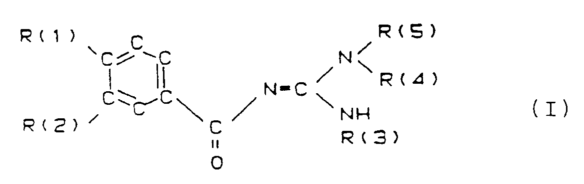 dans laquelle R(1) ou R(2) représente R(6)-S(O)ₙ- ou 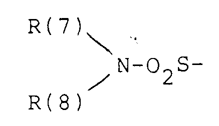 et l'autre substituant R(1) ou R(2) représente H, F, Cl, Br, I, un groupe alkyle en C₁-C₄, alcoxy en C₁-C₄, phénoxy (qui n'est pas substitué ou porte un à trois substituants choisis parmi les atomes de fluor et de chlore et les groupes méthyle et méthoxy ),
R(6)-S(O)ₙ ou 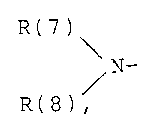 et dans laquelle R(6) représente un groupe alkyle en C₁-C₆, cycloalkyle en C₅-C₇, cyclopentylméthyle, cyclohexylméthyle ou phényle qui n'est pas substitué ou porte un à trois substituants choisis parmi les atomes de fluor et de chlore et les groupes méthyle et méthoxy,
et dans laquelle R(7) et R(8) sont identiques ou différents et représentent H ou un groupe alkyle en C₁-C₆, ou R(7) représente un groupe phényl-(CH₂)ₘ avec m = 1-4, ou phényle qui n'est pas substitué ou porte un ou deux substituants choisis parmi les atomes de fluor ou de chlore et le groupe méthyle ou méthoxy,
et dans laquelle R(7) et R(8) peuvent également être ensemble une chaîne en C₄-C₇ droite ou ramifiée, la chaîne pouvant en outre être interrompue par O, S ou NR(9) et R(9) représentant H ou le groupe méthyle,
et dans laquelle R(7) et R(8) peuvent former ensemble, avec l'atome d'azote auquel ils sont liés, un système dihydroindole, tétrahydroquinoléine ou tétrahydroisoquinoléine,
et R(3), R(4) et R(5) représentent un atome d'hydrogène ou un groupe alkyle en C₁-C₂, R(3) et R(4) pouvant former ensemble une chaîne alkylène en C₂-C₄, et R(4) et R(5) pouvant former ensemble une chaîne alkylène en C₄-C₇,
et n représente zéro, 1 ou 2,
et leurs sels pharmaceutiquement acceptables.

2. Composé I selon la revendication 1, dans lequel R(1) représente un atome de fluor ou de chlore ou un groupe NR(7)R(8), R(6)-S(O)ₙ- ou phénoxy,
R(2) représente un groupe R(6)-S(O)ₙ- ou R(7)R(8)N-SO₂-,
et dans lequel n est zéro, 1 ou 2
et R(3), R(4) et R(5) représentent des atomes d'hydrogène,
et dans lequel R(6), R(7) et R(8) ont les significations données plus haut,
et sels pharmacologiquement acceptables de celui-ci.

3. Composé I selon la revendication 1, dans lequel R(1) représente un groupe R(7)R(8)N- ou R(6)-S(O)ₙ-, R(6) étant un groupe alkyle ayant de 1 à 3 atomes de carbone, ou phényle,
n = zéro,
et R(7) et R(8) représentent un atome d'hydrogène ou un groupe alkyle ayant de 1 à 4 atomes de carbone, ou R(7) et R(8) forment ensemble une chaîne méthylène en C₄-C₇, et R(2) représente CH₃-SO₂- ou H₂N-SO₂- et R(3), R(4) et R(5) représentent des atomes d'hydrogène,
et sels pharmacologiquement acceptables de celui-ci.

4. Composé I selon la revendication 1, caractérisé en ce qu'il est la 3-méthylsulfonyl-4-(1-pipéridinyl)-benzoylguanidine, la 4-phénylthio-3-sulfamoylbenzoylguanidine ou la 3-méthylsulfonyl-4-phénylthiobenzoylguanidine ou un sel pharmacologiquement acceptable.

5. Procédé pour la préparation d'un composé I selon la revendication 1, caractérisé en ce que l'on fait réagir des composés de formule II 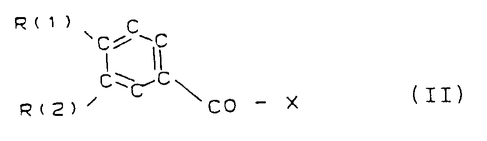 avec un dérivé de guanidine de formule III 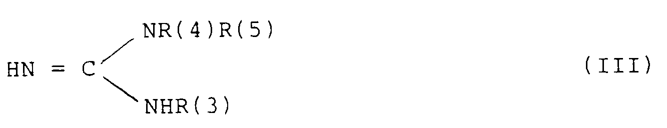 dans lesquelles formule R(1) à R(5) ont les significations données dans la revendication 1, et X représente un groupe partant aisément susceptible de substitution nucléophile.

6. Utilisation d'un composé I selon la revendication 1, pour la fabrication d'un médicament destiné au traitement d'arythmies.

7. Utilisation d'un composé I selon la revendication 1, pour la fabrication d'un médicament destiné au traitement ou à la prophylaxie d'états ischémiques du coeur.

8. Médicament contenant une quantité efficace d'un composé I selon la revendication 1.

## Revendications (Revendications pour l'(les) Etat(s) contractant(s) suivant(s): ES, GR)

1. Procédé pour la préparation d'une benzoylguanidine de formule I 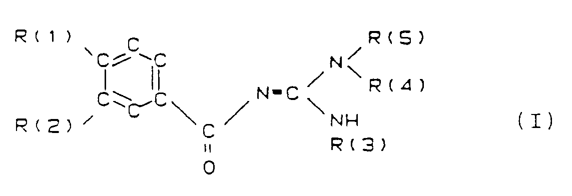 dans laquelle R(1) ou R(2) représente R(6)-S(O)ₙ- ou 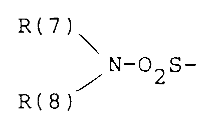 et l'autre substituant R(1) ou R(2) représente H, F, Cl, Br, I, un groupe alkyle en C₁-C₄, alcoxy en C₁-C₄, phénoxy (qui n'est pas substitué ou porte un à trois substituants choisis parmi les atomes de fluor et de chlore et les groupes méthyle et méthoxy),
R(6)-S(O)ₙ ou 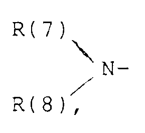 et dans laquelle R(6) représente un groupe alkyle en C₁-C₆, cycloalkyle en C₅-C₇, cyclopentylméthyle, cyclohexylméthyle ou phényle, qui n'est pas substitué ou porte un à trois substituants choisis parmi les atomes de fluor et de chlore et les groupes méthyle et méthoxy,
et dans laquelle R(7) et R(8) sont identiques ou différents et représentent H ou un groupe alkyle en C₁-C₆, ou R(7) représente un groupe phényl-(CH₂)ₘ avec m = 1-4, ou phényle qui n'est pas substitué ou porte un ou deux substituants choisis parmi les atomes de fluor ou de chlore et le groupe méthyle ou méthoxy,
et dans laquelle R(7) et R(8) peuvent également etre ensemble une chaîne en C₄-C₇ droite ou ramifiée, la chaîne pouvant en outre être interrompue par O, S ou NR(9) et R(9) représentant H ou le groupe méthyle,
et dans laquelle R(7) et R(8) peuvent former ensemble, avec l'atome d'azote auquel ils sont liés, un système dihydroindole, tétrahydroquinoléine ou tétrahydroisoquinoléine,
et R(3), R(4) et R(5) représentent un atome d'hydrogène ou un groupe alkyle en C₁-C₂, R(3) et R(4) pouvant former ensemble une chaîne alkylène en C₂-C₄, et R(4) et R(5) pouvant former ensemble une chaîne alkylène en C₄-C₇,
et n représente zéro, 1 ou 2,
ainsi que d'un sel pharmaceutiquement acceptable,
caractérisé en ce que l'on fait réagir un composé de formule II 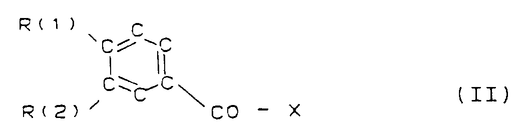 avec un dérivé de guanidine de formule III 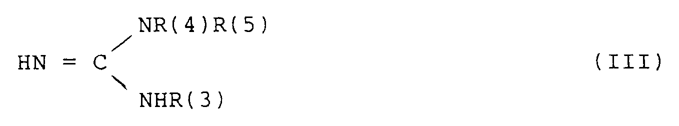 dans lesquelles formule R(1) à R(5) ont les significations données plus haut et X représente un groupe partant aisément susceptible de substitution nucléophile.

2. Procédé selon la revendication 1, caractérisé en ce que l'on utilise des composés II et III dans lesquels les substituants ont les significations suivantes:
R(1) représente un atome de fluor ou de chlore ou un groupe NR(7)R(8), R(6)-S(O)ₙ- ou phénoxy,
R(2) représente un groupe R(6)-S(O)ₙ- ou R(7)R(8)N-SO₂-,
n est zéro, 1 ou 2,
R(3), R(4) et R(5) représentent des atomes d'hydrogène,
R(6), R(7) et R(8) sont tels que définis dans la revendication 1.

3. Procédé selon la revendication 1, caractérisé en ce que l'on utilise des composés II et III dans lesquels:
R(1) représente un groupe R(7)R(8)N- ou R(6)-S(O)ₙ-, R(6) étant un groupe alkyle ayant de 1 à 3 atomes de carbone, ou phényle,
n = zéro,
et R(7) et R(8) représentent un atome d'hydrogène ou un groupe alkyle ayant de 1 à 4 atomes de carbone, ou R(7) et
R(8) forment ensemble une chaîne méthylène en C₄-C₇, et
R(2) représente CH₃-SO₂- ou H₂N-SO₂- et R(3), R(4) et R(5) représentent des atomes d'hydrogène.

4. Procédé selon la revendication 1, caractérisé en ce que l'on prépare les composés 3-méthylsulfonyl-4-(1-pipéridinyl)-benzoyl-guanidine, 4-phénylthio-3-sulfamoylbenzoyl-guanidine ou 3-méthylsulfonyl-4-phénylthiobenzoyl-guanidine ou leurs sels pharmacologiquement acceptables.

5. Procédé pour la fabrication d'un médicament pour le traitement d'arythmies, caractérisé en ce que l'on mélange et met sous une forme appropriée une quantité efficace d'un composé I selon la revendication 1, avec les additifs usuels dans le domaine pharmaceutique.
